# EUROPEAN PATENT APPLICATION

(11) **EP 1 676 566 A1**
(43) Date of publication of application: **05.07.2006**
(21) Application number: 05028574.1
(22) Date of filing: 28.12.2005
(51) Int. Cl.: A61K 8/46, A61K 8/33, A61K 8/39, A61Q 5/02, A61Q 5/12

(54) **Aqueous hair cleansing composition**

(30) Priority: 28.12.2004 JP 2004380016
(71) Applicant: KAO CORPORATION, Tokyo 103-8210 (JP)
(72) Inventor: Terada, Eiji c/o Kao Corporation Research, Sumida-ku Tokyo 131-8501 (JP)
(74) Representative: HOFFMANN EITLE

(57) **Abstract**

Provided is an aqueous hair cleansing composition containing the following components (A) to (C):
(A) from 5 to 30 wt.% of a sulfate surfactant having sulfates represented by the formula: R-O-(C₂H₄O)ₙ-SO₃M (wherein, R represents a C₈₋₁₈ alkyl or alkenyl group, n is 0 or a positive integer and M represents sodium or ammonium) and are composed of from 30 to 45 wt.% of a sulfate with n=0, from 18 to 27 wt.% of a sulfate with n=1, from 10 to 20 wt.% of a sulfate with n=2, and the balance of sulfates with n=3 or greater, and containing the sulfates with n=0 to 2 in a total amount of 70 wt.% or greater based on all the sulfates;
(B) from 0.1 to 15 wt.% of a monoalkyl glyceryl ether or monoalkenyl glyceryl ether having a C₄₋₁₂ alkyl or alkenyl group; and
(C) from 0.1 to 10 wt.% of a silicone oil represented by the formula: R'(CH₃)₂SiO-[(CH₃)₂SiO]ₘ-Si(CH₃)₂R' (wherein, R' represents CH₃ or OH and m is from 50 to 20000) and existing as disperse particles having an average particle size less than 50 µm.

The aqueous hair cleansing composition of the present invention has a good foaming property and very smooth foam quality during shampooing, provides smooth hair feel during rinsing, imparts luster and manageability to hair after drying, has good low-temperature stability and is substantially non-irritating.

## Description

### FIELD OF THE INVENTION

The present invention relates to an aqueous hair cleansing composition containing sulfate surfactant and glyceryl ether.

### BACKGROUND OF THE INVENTION

In recent years, there is a growing need for shampoos having performances such as smoothness of foam during foaming, smoothness of the hair during rinsing and frictionless touch as well as basic performances such as detergency, good foaming property and abundance of foam. Alkyl sulfates typified by sodium dodecyl sulfate have been most popularly employed as an anionic cleansing component for their high detergency and power of producing abundance of foam. When alkyl sulfates are used singly as an anionic component of a cleansing composition, however, feel during shampooing is not satisfactory, because it causes friction between individual hairs. Polyoxyethylene-added alkyl sulfates (alkyl ether sulfates) added with ethylene oxide between the alkyl group and the sulfuric acid group in order to improve the hair feel, particularly those added with 2 to 3 moles, on average, of ethylene oxide have been incorporated in a shampoo because they can impart the hair good feel during shampooing. Addition of ethylene oxide to alkyl sulfates overcomes the problem of friction between individual hairs, but it greatly deteriorates the foaming speed. The above-described method is therefore insufficient for satisfying both good foaming property and reduction in friction.

Alkyl ether sulfates added with 2 moles, on average, of ethylene oxide, which have been typically and conventionally used, are composed of about 20 wt.% of a 0 mole adduct, from 10 to less than 20 wt.% of 1, 2, and 3 mole adducts respectively, and the balance of 4 or greater mole adducts. The foaming property is therefore improved by adjusting the number of moles of ethylene oxide addition. For example, in JP-A-1999-507079, described is an aqueous shampoo composition having a high conditioning effect, which contains from 5 to 50 wt.% of a surfactant component containing alkyl ether sulfates added with 1 to 8 moles of ethylene oxide and an amphoteric surfactant, and containing less than 5 wt.% of alkyl ether sulfates added with not greater than 1 mole of ethylene oxide. This shampoo composition is however unsatisfactory in both foaming property and foam quality.

Further, a silicone oil has recently been added in order to reduce fiction between individual hairs and improve smoothness, but addition of an oil component such as silicone oil to a shampoo composition deteriorates a foaming property and has an influence on a decrease in the foam volume. Therefore such a shampoo composition is unsatisfactory both in foaming property and foam quality.

### SUMMARY OF THE INVENTION

In the present invention, there is provided an aqueous hair cleansing composition containing the following components (A), (B) and (C) :
(A) from 5 to 30 wt.% of a sulfate surfactant having sulfates which are represented by the following formula (1) :

   R-O- (C₂H₄O)ₙ-SO₃M (1)

   (wherein, R represents a linear or branched C₈₋₁₈ alkyl or alkenyl group, n is 0 or a positive integer and M represents sodium or ammonium) and are composed of from 30 to 45 wt.% of a sulfate of the formula (1) in which n=0, from 18 to 27 wt.% of a sulfate of the formula (1) in which n=1, from 10 to 20 wt.% of a sulfate of the formula (1) in which n=2, and the balance of sulfates of the formula (1) in which n=3 or greater, and containing the sulfates of the formula (1) in which n=0 to 2 in a total amount of 70 wt.% or greater based on all the sulfates;
(B) from 0.1 to 15 wt.% of a monoalkyl glyceryl ether or monoalkenyl glyceryl ether having a C₄₋₁₂ alkyl or alkenyl group; and
(C) from 0.1 to 10 wt.% of a silicone oil represented by the following formula (2):

   R' (CH₃)₂SiO- [(CH₃)₂SiO]ₘ-Si(CH₃)₂R' (2)

   (wherein, R' represents a methyl or hydroxy group and m is a number of from 50 to 20000) and existing as disperse particles having an average particle size less than 50 µm.

### DETAILED DESCRIPTION OF THE INVENTION

The present invention relates to an aqueous hair cleansing composition which has a good foaming property and a very smooth foam quality during shampooing, provides smooth hair feel during rinsing, gives luster and manageability to hair after drying, has good low-temperature stability and is substantially non-irritating.

The present inventors have found that a hair cleansing composition satisfying the above-described demand is obtainable by using a sulfate component containing polyoxyethylene added alkyl sulfates which are distributed on the smaller side of the number of moles of ethylene oxide addition, that is, a sulfate component having a percentage of the sulfates added with 0 to 2 moles of ethylene oxide falling within a predetermined range; a monoalkyl glyceryl ether or monoalkenyl glyceryl ether having a C₄₋₁₂ alkyl or alkenyl group; and a silicone oil having a small particle size.

The sulfate surfactant serving as Component (A) of the present invention is represented by the following formula (1):

R-O-(C₂H₄O)ₙ-SO₃M (1)

In the present invention, good foam quality, abundant foam volume and fine hair feel can be attained by the sulfate surfactant having a specific distribution in the number of moles of ethylene oxide addition and having a high percentage of sulfates whose number of moles of EO addition is small, more specifically, the sulfate surfactant having a distribution which is composed of from 30 to 45 wt.% of a sulfate of the formula (1) in which n=0, from 18 to 27 wt.% of a sulfate of the formula (1) in which n=1, from 10 to 20 wt.% of a sulfate of the formula (1) in which n=2 and the balance of sulfates of the formula (1) in which n≥3 and containing the sulfates of the formula (1) in which n=0 to 2 in a total amount of 70 wt.% or greater based on all the sulfates. The sulfate surfactant having such a sulfate distribution is preferably composed of sulfates equal in alkyl chain length and only different in the number of moles of ethylene oxide addition.

In order to satisfy both quick foaming and fine feel of foam, the sulfate surfactant having the following distribution in the number of moles of ethylene oxide addition, that is, the sulfate surfactant composed of from 33 to 43 wt.% of a sulfate with n=0, from 20 to 25 wt.% of a sulfate with n=1, from 13 to 18 wt.%, of a sulfate with n=2 and the balance of sulfates with n≥3 is preferred, in which that composed of from 35 to 41 wt.% of a sulfate with n=0, from 21 to 23 wt.% of a sulfate with n=1, from 14 to 17 wt.% of a sulfate with n=2 and the balance of sulfates with n≥3 is more preferred. From the same viewpoint, the percentage of the sulfates with n= 0 to 2 in the sulfate surfactant component is 70 wt.% or greater, with 70 to 85 wt.% based on all the sulfates being more preferred.

Such a sulfate surfactant can be prepared, for example, by sulfating an alcohol ethoxylate, which has been obtained by adding from 0.85 to 1.35 times the mole of ethylene oxide to a higher alcohol ROH, with from 0.95 to 1.0 equivalent of SO₃ and then neutralizing the resulting sulfate with sodium hydroxide or ammonia. In the formula (1), M is preferably ammonium from the viewpoint of fine feel of foam.

The content of the sulfate surfactant as Component (A) in the aqueous hair cleansing composition of the present invention is from 5 to 30 wt.%. From the viewpoints of good foaming property, abundant foams and foam quality with good smoothness during shampooing, it is preferably from 7 to 23 wt.%, more preferably from 10 to 20 wt.%.

Component (B) of the present invention is a monoalkyl glyceryl ether or monolakenyl glyceryl ether having a C₄₋₁₂ alkyl or alkenyl group. Monoalkyl glyceryl ethers having a linear or branched C₈₋₁₀ alkyl group are preferred. Of these, a monoalkyl glyceryl ether having a n-butyl, isobutyl, n-pentyl, 2-methylbutyl, isopentyl, n-hexyl, isohexyl, n-heptyl, n-octyl, 2-ethylhexyl, n-decyl or isodecyl group is preferred, with a monoalkyl glyceryl ether having a 2-ethylhexyl or isodecyl group being more preferred

As Component (B), two or more of these compounds may be used in combination. The content thereof is from 0.1 to 15 wt.% in the hair cleansing composition of the present invention. The content is preferably from 0.2 to 10 wt.%, more preferably from 0.3 to 5 wt.% because it impairs neither feeling upon use nor conditioning effects and contributes to producing more sufficient foaming power.

The silicone oil serving as Component (C) is represented by the formula (2) and exists as disperse particles having an average particle size less than 50 µm. It is substantially water insoluble and nonvolatile. Component (C) is preferably a water-insoluble highly-polymerized dimethylpolysiloxane emulsion obtained by emulsion polymerization.

The average particle size of the silicone oil as Component (C) is less than 50 µm, preferably not greater than 4 µm, more preferably not greater than 3 µm, still more preferably not greater than 2 µm. In order to attain good feeling upon use and conditioning effects, however, the average particle size is preferably 0.1 µm or greater.

As such a silicone oil, a silicone emulsion commercially available as "Silicone CF2450" from Dow Corning Toray which contains 60 wt.% of a dimethylpolysiloxane oil of the formula (2) in which m stands for 300 to 6500 and has an average particle size of 0.5 µm can be used, for example.

The silicone oil having such a small particle size as Component (C) is incorporated in an amount of from 0.01 to 10 wt.%, preferably from 0.05 to 6 wt.%, more preferably from 0.5 to 3 wt.%, still more preferably from 1 to 2 wt.% in the aqueous hair cleansing composition of the present invention in order to improve the feel of foam during shampooing and the hair feel and luster after drying.

For the aqueous hair cleansing composition of the present invention, one or more than one nonionic surfactants or amphoteric surfactants other than Component (B) can be used in combination in order to improve its detergency.

Examples of the nonionic surfactants other than Component (B) include polyoxyalkylene sorbitan fatty acid esters, polyoxyalkylene sorbitol fatty acid esters, polyoxyalkylene glycerol fatty acid esters, polyoxyalkylene fatty acid esters, polyoxyalkylene alkyl ethers, polyoxyalkylene alkylphenyl ethers, polyoxyalkylene (hydrogenated) castor oils, sucrose fatty acid esters, polyglyceryl alkyl ethers, polyglyceryl fatty acid esters, fatty acid alkanolamides, and alkyl glycosides. Of these, alkyl glycosides, polyoxyalkylene (C₈-C₂₀) fatty acid esters, polyoxyethylene sorbitan fatty acid esters, polyoxyethylene hydrogenated castor oil and fatty acid alkanolamides are preferred.

The fatty acid alkanolamides may be either monoalkanolamides or dialkanolamides. Those having a C₈₋₁₈ acyl group are preferred, with those having a C₁₀₋₁₆ acyl group being more preferred. Those having a C₂₋₃ hydroxyalkyl group are more preferred. Examples include oleic diethanolamide, palm kernel oil fatty acid diethanolamide, coconut oil fatty acid diethanolamide, lauric diethanolamide, polyoxyethylene coconut oil fatty acid monoethanolamide, coconut oil fatty acid monoethanolamide, lauric isopropanolamide, and lauric monoethanolamide.

Examples of the amphoteric surfactant include betaine surfactants. Of these, betaine surfactants such as alkyldimethylaminoacetic acid betaines, fatty acid amidopropylbetaines and alkylhydroxysulfobetaines are more preferred, of which fatty acid amidopropylbetaines are more preferred. The fatty acid amidopropylbetaines preferably have a C₈₋₁₈ acyl group, more preferably a C₁₀₋₁₆ acyl group. Preferred specific examples include lauric acid amidopropyl betaine, palm kernel oil fatty acid amidopropylbetaine and coconut oil amidopropylbetaine.

In the aqueous hair cleansing composition of the present invention, these nonionic or amphoteric surfactants other than Component (B) can be used either singly or in combination of two or more. In order to formulate the aqueous hair cleansing composition of the present invention into an aqueous liquid cleansing composition, use of a fatty acid alkanolamide or fatty acid amidopropylbetaine as a surfactant other than Components (A) and (B) is desired because this makes it possible not only to further improve the foaming power but also to give the agent with adequate liquid properties.

The content of the nonionic or amphoteric surfactant other than Component (B) is from 0.1 to 10 wt.% in the hair cleansing composition of the present invention, but is preferably from 0.5 to 8 wt.%, more preferably from 1 to 6 wt.% in order to increase the foam volume.

In the present invention, one or more anionic surfactants other than the sulfate surfactant may be added. For example, sulfonate surfactants and carboxylate surfactants can be used. Examples include alkyl sulfosuccinates, polyoxyalkylene alkyl sulfosuccinates, higher fatty acid salts, alkanesulfonates, and alkyl ether carboxylic acids or salts thereof.

Furthermore, the aqueous hair cleansing composition of the present invention may contain a cationic polymer from the viewpoints of the texture and smoothness of foam, reduction in friction among individual hairs during cleansing and smoothness of hair during drying. Specific examples of the cationic polymer include cationic cellulose derivatives, cationic starch, cationic guar gum derivatives, homopolymers of a diallyl quaternary ammonium salt, diallyl quaternary ammonium salt/acrylamide copolymers, quaternized polyvinylpyrrolidone derivatives, polyglycol polyamine condensates, vinyl imidazolium trichloride/vinylpyrrolidone copolymer, hydroxyethyl cellulose/dimethyldiallylammonium chloride copolymer, vinylpyrrolidone/quaternized dimethylaminoethyl methacrylate copolymers, polyvinylpyrrolidone/alkylaminoacrylate copolymers, polyvinylpyrrolidone/alkylaminoacrylate/vinylcaprolactam copolymers, vinylpyrrolidone/methacrylamidopropyl trimethylammonium chloride copolymer, alkylacrylamide/acrylate/alkylaminoalkylacrylamide/polyethy leneglycol methacrylate copolymers, adipic acid/dimethylaminohydroxypropyl ethylenetriamine copolymer ("Cartaretin", trade mark; product of Sandoz/USA), and cationic polymers as described in JP-A-1978-139734 and JP-A-1985-36407. Of these, cationic cellulose derivatives, cationic guar gum derivatives, and diallyl quaternary ammonium salt/acrylamide copolymers are preferred.

As the cationic polymer, two or more of these compounds may be used in combination. The content thereof in the aqueous hair cleansing composition of the present invention is preferably from 0.02 to 5 wt.%, more preferably from 0.05 to 1 wt.%, still more preferably from 0.1 to 0.7 wt.% in order to improve the foam quality during cleansing, and manageability and hair feel after drying.

To the aqueous hair cleansing composition of the present invention, a conditioning component such as cationic surfactant and silicone may be added further for improving the finish of the hair after drying.

Examples of the cationic surfactant include alkyltrimethylammonium salts, alkoxytrimethylammonium salts, dialkyldimethylammonium salts, alkyldimethylamines and salts thereof, alkoxydimethylamines and salts thereof, and alkylamidodimethylamines and salts thereof.
(i) Alkyltrimethylammonium salts
   They are, for example, represented by the following formula:

   R¹¹-N⁺(Me)₃X⁻

   (wherein, R¹¹ represents a C₁₂₋₂₂ alkyl group, Me represents a methyl group, and X⁻ represents a halogen (chlorine or bromine) ion or a C₁₋₂ alkylsulfate ion).
(ii) Alkoxytrimethylammonium salts
   They are, for example, represented by the following formula:

   R¹²-O-R¹³-N⁺(Me)₃X⁻

   (wherein, R¹² represents a C₁₂₋₂₂ alkyl group, R¹³ represents an ethylene or propylene group, Me represents a methyl group, and X⁻ has the same meaning as described above).
(iii) Dialkyldimethylammonium salts
   They are, for example, represented by the following formula:

   R¹⁴₂-N⁺(Me)₂X⁻

   (wherein, R¹⁴ represents a C₁₂₋₂₂ alkyl group or a benzyl group, Me represents a methyl group, and X⁻ has the same meaning as described above).
(iv) Alkyldimethylamines and salts thereof
   They are, for example, represented by the following formula:

   R¹⁵-N(Me)₂

   (wherein, R¹⁵ represents a C₁₂₋₂₂ alkyl group, and Me represents a methyl group).
(v) Alkoxydimethylamines and salts thereof
   They are, for example, represented by the following formula:

   R¹⁶-O-R¹⁷-N(Me)₂

   (wherein, R¹⁶ represents a C₁₂₋₂₂ alkyl group, R¹⁷ represents an ethylene or propylene group and Me represents a methyl group).
(vi) Alkylamidodimethylamines and salts thereof
   They are, for example, represented by the following formula:

   R¹⁸-C(=O)NH-R¹⁹-N(Me)₂

   (wherein, R¹⁸ represents a C₁₁₋₂₁ alkyl group, R¹⁹ represents an ethylene or propylene group and Me represents a methyl group) .

Examples of the cationic surfactant other than those described in (i) to (vi) include lanolin fatty acid aminopropylethyldimethylammonium ethyl sulfate (ethyl sulfate salt of alkanoylaminopropyldimethylethylammonium, said alkanoyl group being derived from lanolin), lanolin fatty acid aminoethyltriethylammonium ethyl sulfate, lanolin fatty acid aminopropyltriethylammonium ethyl sulfate, lanolin fatty acid aminoethyltrimethylammonium methyl sulfate, lanolin fatty acid aminopropylethyldimethylammonium methyl sulfate, isoalkanoic acid (C₁₄-C₂₀) aminopropylethyldimethylammonium ethyl sulfate, isoalkanoic acid (C₁₈-C₂₂) aminopropylethyldimethylammonium ethyl sulfate, isostearic acid aminopropylethyldimethylammonium ethyl sulfate, isononanoic acid aminopropylethyldimethylammonium ethyl sulfate, and alkyltrimethylammonium saccharins.

These cationic surfactants may be used in combination of two or more. The content thereof is preferably from 0.01 to 10 wt.%, more preferably from 0.05 to 6 wt.%, still more preferably from 0.3 to 3 wt.%, still more preferably from 0.5 to 2 wt.% in the aqueous hair cleansing composition of the present invention from the viewpoint of smoothness from shampooing throughout rinsing.

Silicones other than Component (C) are, for example, those as described below.

### (1) Dimethylpolysiloxane

As dimethylpolysiloxane other than Component (C), either volatile dimethylpolysiloxane or low-molecular-weight liquid nonvolatile dimethylpolysiloxane may be used in combination. Commercially available products include "SH200" (trade name; product of Dow Corning Toray) and "KF96" (trade name; product of Shin-etsu Chemical).

### (2) Amino-modified silicones

Various amino-modified silicones can be used. Those having an average molecular weight from about 3000 to 100000 and listed under the name of "Amodimethicone" in the third edition of CTFA Dictionary (Cosmetic Ingredient Dictionary/USA) are preferred. These amino-modified silicones are preferably employed as an aqueous emulsion. Commercially available products include "SM 8704C" (trade name; product of Dow Corning Toray), "DC 929" (trade name; product of Dow Corning) and "KT1989" (trade name; product of GE Toshiba Silicones).

### (3) Other silicones

Examples of the silicone other than those described above include polyether modified silicones, methylphenylpolysiloxane, fatty acid modified silicones, alcohol modified silicones, alkoxy modified silicones, epoxy modified silicones, fluorine modified silicones, cyclic silicones and alkyl modified silicones.

Two or more of these silicones may be used in combination. The content thereof in the aqueous hair cleansing composition of the present invention is preferably from 0.01 to 10 wt.%, more preferably from 0.05 to 6 wt.%, still more preferably from 0.3 to 3 wt.%, still more preferably from 0.5 to 2 wt.% from the viewpoint of the smoothness from shampooing through rinsing.

The aqueous hair cleansing composition of the present invention may contain an oil agent as another conditioning agent. Examples of the oil agent include hydrocarbons such as squalene, squalane, liquid paraffin, liquid isoparaffin, and cycloparaffin; glycerides such as castor oil, cacao oil, mink oil, avocado oil, and olive oil; waxes such as beeswax, whale wax, lanolin, and carnauba wax; esters such as isopropyl palmitate, isopropyl myristate, octyldodecyl myristate, hexyl laurate, cetyl lactate, propylene glycol monostearate, oleyl oleate, hexadecyl 2-ethylhexanoate, isononyl isononanoate and tridecyl isononanoate; higher fatty acids such as capric acid, lauric acid, myristic acid, palmitic acid, stearic acid, behenic acid, oleic acid, coconut oil fatty acids, isostearic acid, and isopalmitic acid; higher alcohols such as myristyl alcohol, cetyl alcohol, oleyl alcohol, stearyl alcohol, isostearyl alcohol, behenyl alcohol, 2-octyldodecanol and cetostearyl alcohol; isostearyl glyceryl ether; and polyoxypropylene butyl ether. Of these, higher alcohols, for example, myristyl alcohol, cetyl alcohol and stearyl alcohol are preferred. These oil agents may be use either singly or in combination of two or more. The content thereof is preferably from 0.2 to 2 wt.%, more preferably from 0.3 to 1.8 wt.%, still more preferably from 0.5 to 1.5 wt.% in the aqueous hair cleansing composition of the present invention.

In addition, a pearling agent containing an ethylene glycol monoalkyl ester or ethylene glycol dialkyl ester may be incorporated in the cleansing composition to improve its texture and stability. Examples of the ethylene glycol monoalkyl ester include ethylene glycol monostearyl ester and ethylene glycol monobehenyl ester, while those of the ethylene glycol dialkyl ester include ethylene glycol distearyl ester and ethylene glycol dibehenyl ester. Two or more of them may be used in combination. The content of the pearling agent in the aqueous hair cleansing composition of the present invention is preferably from 0.5 to 8 wt.%, more preferably from 0.7 to 5 wt.%, still more preferably from 1 to 3 wt.%. In view of improving stability of the cleansing composition, the weight ratio of the pearling agent to the sulfate surfactant (pearling agent/sulfate surfactant) contained in the hair cleansing composition of the present invention is preferably from 1/10 to 2/5, more preferably from 1/7 to 3/10, still more preferably from 1/6 to 1/4.

The aqueous hair cleansing composition of the present invention may contain a viscosity regulator. Examples of the viscosity regulator include hydroxyethyl cellulose, methyl cellulose, polyethylene glycol, polypropylene glycol, ethylene glycol, propylene glycol, isoprene glycol, ethanol, benzyl alcohol, benzyl oxyethanol, phenoxyethanol, clay minerals, and salts (sodium chloride, ammonium chloride, sodium citrate and the like). Of these, salts are preferred, with sodium chloride and sodium citrate being more preferred. Two or more of the viscosity regulators may be used in combination. The content thereof in the aqueous hair cleansing composition of the present invention is preferably from 0.01 to 5 wt.%, more preferably from 0.05 to 3 wt.%, still more preferably from 0.1 to 1.5 wt.% from the standpoints of the volume and quality of foam.

In addition to the above-described components, components which are employed in ordinary hair cleansing compositions can also be incorporated in the aqueous hair cleansing composition of the present invention as needed depending upon the purpose of use. Such components include, for example, antidandruff agents; vitamin preparations; bactericides; anti-inflammatories; antiseptics; chelating agents; humectants such as sorbitol, panthenol and glycerin; colorants such as dyes and pigments; extracts such as extracts of eucalyptus in a polar solvent, proteins available from shells having a pearl layer or pearls or hydrolysates thereof, proteins available from silk or hydrolysates thereof, protein-containing extracts available from seeds of legume, Asian ginseng extract, rice bran extract, fucus vesiculosus extract, aloe extract, Alpinia Leaf extract, and chlorella extract; pearling agents such as titanium oxide; perfumes; coloring matters; ultraviolet absorbers; antioxidants; and other components described in ENCYCLOPEDIA OF SHAMPOO INGREDIENTS (MICELLE PRESS).

The aqueous hair cleansing composition of the present invention has preferably pH (diluted with 20 times the amount of water, 25°C) of from 2 to 6, more preferably from 3 to 5, still more preferably from 3.5 to 4.5 when it is applied to the hair, from the viewpoint of improving the luster and manageability of the hair. As a pH regulator, an organic acid is preferred, with an α-hydroxy acid being more preferred. Specific preferred examples of it include malic acid, citric acid, lactic acid and glycolic acid. As the pH regulator, two or more of these organic acids may be used in combination. The amount of use thereof is preferably from 0.01 to 5 wt.%, more preferably from 0.1 to 3 wt.%, still more preferably from 0.3 to 2 wt.% in the aqueous hair cleansing composition of the present invention from the viewpoints of improvement in foam quality and flexibility of hair during shampooing. As another pH regulator, a base such as sodium hydroxide, potassium hydroxide or ammonium chloride may be used in combination with the above-described organic acid.

Although the form of the aqueous hair cleansing composition of the present invention can be chosen as desired from a liquid form, a gel form and the like, a liquid form in a solvent such as water or a lower alcohol is preferred, with the former one being more preferred.

### -Examples-

### Preparation Example 1: Preparation of ammonium lauryl ether sulfate added with 1.0 mole, on average, of EO

In a pressure-tolerant hermetically-sealed reactor were charged with 2000 g of "Kalcol 2470" (trade name; product of Kao, dodecyl alcohol:tetradecyl alcohol = about 3:1) and 1.45 g of potassium hydroxide. After dehydration at 110°C and 10 mmHg for 30 minutes, the temperature in the system was raised to 165°C. Then, 456 g of ethylene oxide was pressed into the reactor and the addition reaction was conducted for 30 minutes without changing the temperature. The reaction mixture was then cooled to 80°C, and neutralized with 1.3 g of acetic acid, whereby an ethylene oxide adduct of the above-described raw material alcohol was obtained.

A sulfation reaction was then effected at 40°C by using 1793 g of the mixture obtained by the above-described operation and 607 g of a sulfuric acid gas. After completion of the reaction, the reaction mixture was neutralized with 150 g of 28 wt.% aqueous ammonia and 600 g of deionized water. The concentration and pH of the neutralized mixture were adjusted further with 28 wt.% aqueous ammonia and deionized water, whereby 10000 g of Sulfate 1 (a 25 wt.% aqueous solution) as shown in Table 1 was obtained.

The sulfate, anion and EO chain of the resulting Sulfate 1 were confirmed in accordance with the Japanese Standards of Cosmetic Ingredients, while a component ratio was analyzed by gas chromatography. They are shown in Table 1.

### Preparation Example 2: Preparation of ammonium lauryl ether sulfate added with 1.3 moles, on average, of EO

In a similar manner to that employed in Preparation Example 1 except for a change in the reaction ratio of the raw materials, Sulfate 2 (a 25 wt.% aqueous solution) as shown in Table 1 was obtained.

The sulfate, anion and EO chain of the resulting Sulfate 2 were confirmed in accordance with the Japanese Standards of Cosmetic Ingredients, while a component ratio was analyzed by gas chromatography. They are shown in Table 1.

### Preparation Example 3: Preparation of sodium lauryl ether sulfate added with 1.0 mole, on average, of EO

A sulfation reaction was effected at 40°C by using 1793 g of the ethylene oxide adduct of the raw material alcohol obtained in Preparation Example 1 and 607 g of a sulfuric acid gas. After completion of the reaction, the reaction mixture was neutralized with 132 g of a 23 wt.% aqueous solution of sodium hydroxide and 556 g of deionized water. The concentration and pH of the neutralized mixture were adjusted further with a 23 wt.% aqueous solution of sodium hydroxide, 75 wt.% of phosphoric acid and deionized water, whereby 10000 g of Sulfate 3 (a 25 wt.% aqueous solution) as shown in Table 1 was obtained.

The sodium salt, sulfate, anion and EO chain of the resulting Sulfate 3 were confirmed in accordance with the Japanese Standards of Cosmetic Ingredients, while a component ratio was analyzed by gas chromatography. They are shown in Table 1.

**Table 1**

| (wt.%) | | | | | |
|---|---|---|---|---|---|
| | n=0 | n=1 | n=2 | n=3 | n≥4 |
| Sulfate 1 of Preparation Example 1 Ammonium lauryl ether sulfate added with 1.0 mole, on average, of EO | 40.64 | 22.29 | 14.80 | 8.68 | 13.59 |
| Sulfate 2 of Preparation Example 2 Ammonium lauryl ether sulfate added with 1.3 moles, on average, of EO | 34.29 | 21.41 | 16.59 | 10.09 | 17.62 |
| Sulfate 3 of Preparation Example 3 Sodium lauryl ether sulfate added with 1.0 mole, on average, of EO | 40.64 | 22.29 | 14.80 | 8.68 | 13.59 |
| Comparative Sulfate 1 *¹ Sodium lauryl ether sulfate added with 2.0 moles, on average, of EO | 19.97 | 15.99 | 16.03 | 13.20 | 34.82 |
| Comparative Sulfate 2 *² Mixture of sodium lauryl ether sulfate added with 2.0 moles, on average, of EO ("Emal 227-PH11") and sodium lauryl sulfate | 46.43 | 10.70 | 10.73 | 8.83 | 23.31 |

| | | | | | |
|---|---|---|---|---|---|
| *1: Comparative Sulfate 1 "Emal 227-PH11" of Kao which is a 27 wt.% aqueous solution of sulfate (sodium lauryl ether sulfate added with 2.0 moles, on average, of EO) | | | | | |
| *2: Comparative Sulfate 2 Mixture of sodium lauryl ether sulfate added with 2.0 moles, on average, of EO and sodium lauryl sulfate. A 27 wt.% aqueous solution of the sulfate was obtained by mixing 1000 g of "Emal 227-PH11" and 500 g of "Emal 2F-HP", each product of Kao. | | | | | |

### Examples 1 to 7, and Comparative Examples 1 to 3

Hair cleansing compositions as shown in Table 2 were prepared using the sulfate surfactants as shown in Table 1 and foaming speed, smoothness of the hair when shampooed with them, and luster and manageability of the hair after drying were evaluated. The pH is a value as measured at 25°C after dilution with 20 times the amount of water.

### (Foaming speed)

By employing the apparatus and conditions as described in [0060] and [0061] of JP-A-1998-73584, the foam volume thus produced was measured using 1.5 mL of a sample to be evaluated and 0.3 mL of a model sebum and the foaming speed was evaluated based on the time until the foam volume reached 25 mL.

### Criteria for evaluation:

A: less than 100 seconds
B: 100 seconds or greater but less than 200 seconds
C: 200 seconds or greater but less than 300 seconds
D: 300 seconds or greater

### (Smoothness of the hair during shampooing)

After a human hair bundle of 25 cm in length, 5.5 cm in width and 10 g in weight was rinsed lightly with warm water of 40°C, excess water was removed. The hair cleansing composition (0.5 g) was foamed sufficiently for about 30 seconds to shampoo the hair bundle and the smoothness of the hair with foam was organoleptically evaluated. Evaluation was carried out by a panel of five experts and their total scores were indicated.

### Criteria for evaluation:

4: Very smooth
3: Smooth
2: not so smooth
1: Not smooth

### (Luster and manageability after drying)

A hair bundle treated in a similar manner to that employed in the evaluation of smoothness was rinsed for 30 seconds with running water (2 L/min) of 40°C and then towel-dried sufficiently. After natural drying, the luster and manageability were evaluated visually. They were evaluated by a panel of 5 experts and their total scores were indicated.

### Criteria for evaluation:

4: Very good
3: Good
2: Not so good
1: Not good

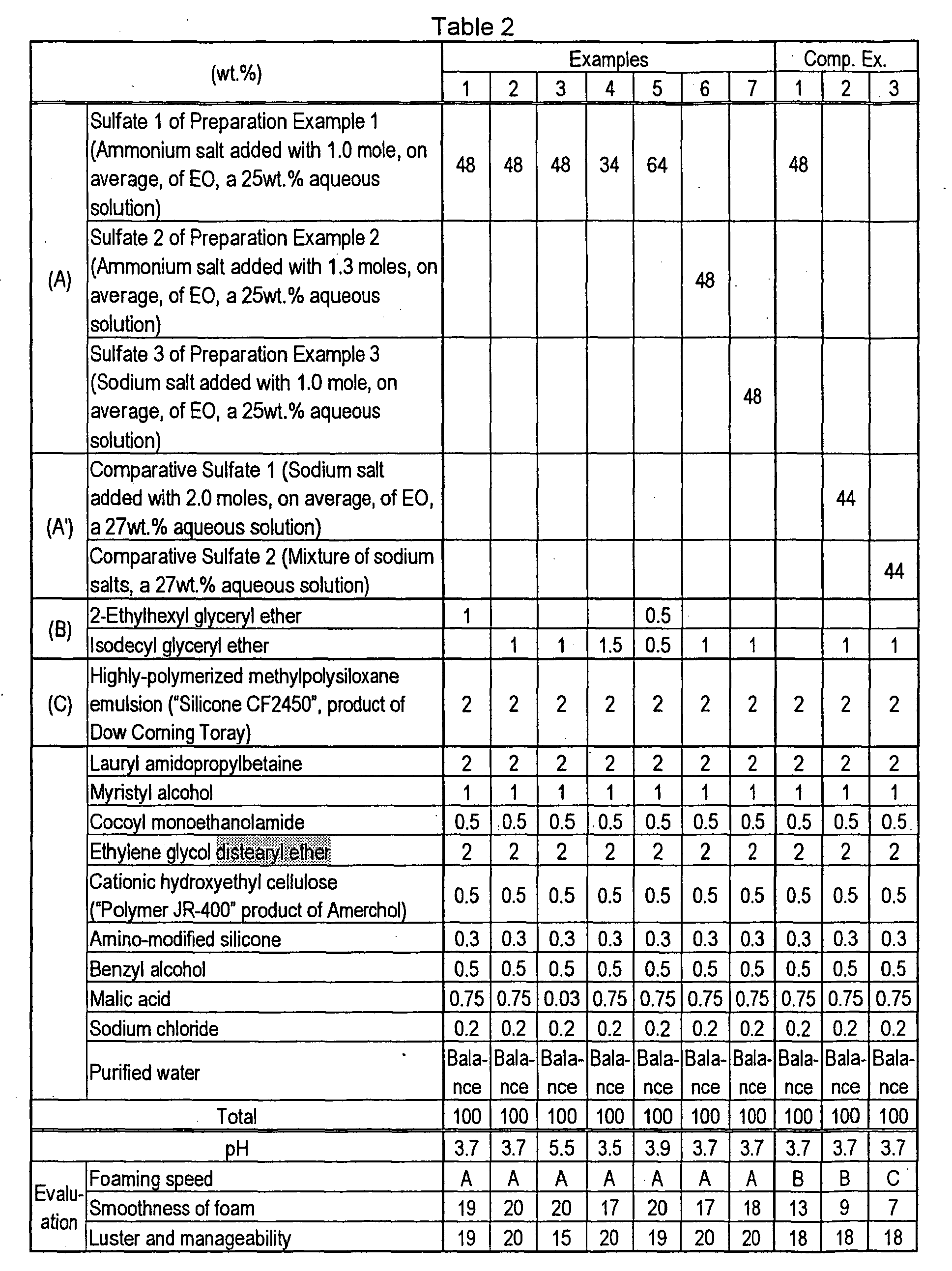

### Example 8: Pearlescent shampoo

| | (wt.%) |
|---|---|
| Sulfate 3 (sodium salt, a 25 wt.% aqueous solution) obtained in Preparation Example 3 | 48.0 |
| 2-Ethylhexyl glyceryl ether | 1.5 |
| Lauryl amidopropylbetaine | 2.0 |
| Sodium cocoamphoacetate | 0.5 |
| Cocoyl monoethanolamide | 0.8 |
| Polyoxyethylene (14) lauryl ether | 1.0 |
| Ethylene glycol distearate | 2.0 |
| Cationic hydroxyethyl cellulose ("Polymer JR-400", trade name; product of Amerchol") | 0.2 |
| Cationic guar gum ("Jaguar C-13S", trade name; product of Rhodia) | 0.2 |
| Highly polymerized methylpolysiloxane emulsion ("Silicone CF2450", trade name; product of Dow Corning Toray) | 1.5 |
| Propylene glycol | 0.5 |
| Panthenol | 0.05 |
| Silk extract | 0.05 |
| Sodium chloride | 1.0 |
| Perfume | q.s. |
| Lactic acid | Amount to adjust pH to 6.0 |
| Deionized water | Balance |

### Example 9: Conditioning shampoo

| | (wt.%) |
|---|---|
| Sulfate salt 1 (ammonium salt, a 25 wt.% aqueous solution) obtained in Preparation Example 1 | 52.0 |
| Isodecyl glyceryl ether | 0.7 |
| Lauryl amidopropylbetaine | 2.0 |
| Cocoyl monoethanolamide | 0.5 |
| Myristyl alcohol | 1.5 |
| Polyoxyethylene (16) lauryl ether | 1.0 |
| Ethylene glycol distearate | 2.0 |
| Cationic hydroxyethyl cellulose ("Poiz C-80M", trade name; product of Kao) | 0.5 |
| Diallyl quaternary ammonium salt/acrylamide copolymer ("Merquat 550", trade name; product of Calgon) | 0.2 |
| Amino-modified silicone ("SM8704C", trade name; product of Dow Corning Toray) | 1.2 |
| Highly polymerized methylpolysiloxane emulsion ("Silicone CF2450", trade name; product of Dow Corning Toray) | 1.5 |
| Benzyl alcohol | 0.4 |
| Polypropylene glycol (Mw=400) | 0.2 |
| Sodium chloride | 1.0 |
| Hydrolyzed conchiolin solution (dry content: 3 wt.%) | 0.05 |
| Asian ginseng extract (dry content: 3 wt.%) | 0.05 |
| Soybean extract (dry content: 0.4 wt.%) | 0.05 |
| Eucalyptus extract (dry content: 0.2 wt.%) | 0.05 |
| Rice bran oil | 0.05 |
| Malic acid | 0.5 |
| Perfume | q.s. |
| Sodium hydroxide | Amount to adjust pH to 3.9 |
| Deionized water | Balance |

### Example 10: Conditioning shampoo

| | (wt.%) |
|---|---|
| Sulfate salt 1 (ammonium salt, a 25 wt.% aqueous solution) obtained in Preparation Example 1 | 64.0 |
| Isodecyl glyceryl ether | 0.3 |
| Cocoyl monoethanolamide | 0.5 |
| Myristyl alcohol | 1.0 |
| Cetanol | 0.5 |
| Ethylene glycol distearate | 3.0 |
| Cationic hydroxyethyl cellulose ("Polymer JR-30M, trade name; product of Amerchol) | 0.2 |
| Cationic guar gum ("Jaguar C-13S", trade name; product of Rhodia) | 0.3 |
| Glycerin | 1.0 |
| Highly polymerized methylpolysiloxane emulsion ("Silicone CF2450", trade name; product or Dow Corning Toray) | 0.5 |
| Sodium chloride | 0.2 |
| Benzyloxyethanol | 0.5 |
| Malic acid | 0.7 |
| Perfume | q.s. |
| Lactic acid | Amount to adjust pH to 3.7 |
| Deionized water | Balance |

### Example 11: Conditioning shampoo

| | (wt.%) |
|---|---|
| Sulfate salt 2 (ammonium salt, a 25 wt.% aqueous solution) obtained in Preparation Example 2 | 40.0 |
| 2-Ethylhexyl glyceryl ether | 1.0 |
| Lauryl amidopropylbetaine | 3.0 |
| Polyoxyethylene (16) lauryl ether | 2.0 |
| Stearoxypropyldimethylamine·malate | 0.5 |
| Ethylene glycol distearate | 2.0 |
| Cationic guar gum ("Jaguar C-13S", trade name; product of Rhodia) | 0.3 |
| Polypropylene glycol (Mw=400) | 0.5 |
| Sodium chloride | 1.0 |
| Malic acid | 0.8 |
| Glycolic acid | 0.75 |
| Highly polymerized methylpolysiloxane emulsion ("Silicone CF2450", product or Dow Corning Toray) | 4.0 |
| Sodium hydroxide | Amount to adjust pH to 3.5 |
| Deionized water | Balance |

### Example 12: Pearlescent anti-dandruff shampoo

| | (wt.%) |
|---|---|
| Sulfate salt 1 (ammonium salt, a 25 wt.% aqueous solution) obtained in Preparation Example 1 | 52.0 |
| Isodecyl glyceryl ether | 1.5 |
| Cocoyl amidopropylbetaine | 1.0 |
| Lauryl hydroxysulfobetaine | 1.0 |
| Lauric acid | 0.5 |
| Oleic acid' | 0.7 |
| Distearyl ether | 2.0 |
| Cocoyl benzalkonium chloride | 0.5 |
| Cationic hydroxyethyl cellulose ("Polymer JR-400, trade name; product of Amerchol) | 0.4 |
| Ethanol | 0.5 |
| Highly polymerized methylpolysiloxane emulsion ("Silicone CF2450", trade name; product or Dow Corning Toray) | 2.0 |
| Fucus vesiculosus extract | 0.05 |
| Malic acid | 0.7 |
| Perfume | q.s. |
| Sodium hydroxide | Amount to adjust pH to 5.5 |
| Deionized water | Balance |

The hair cleansing compositions obtained in Examples 8 to 12 featured speedy foaming and smooth feel of foam and at the same time, gave good luster and manageability to the hair after drying.

## Claims

1. An aqueous hair cleansing composition comprising the following components (A), (B) and (C):
(A) from 5 to 30 wt.% of a sulfate surfactant having sulfates which are represented by the following formula (1):
R-O-(C₂H₄O)ₙ-SO₃M (1)
(wherein, R represents a linear or branched C₈₋₁₈ alkyl or alkenyl group, n is 0 or a positive integer and M represents sodium or ammonium) and are composed of from 30 to 45 wt.% of a sulfate of the formula (1) in which n=0, from 18 to 27 wt.% of a sulfate of the formula (1) in which n=1, from 10 to 20 wt.% of a sulfate of the formula (1) in which n=2, and the balance of sulfates of the formula (1) in which n=3 or greater, and containing the sulfates of the formula (1) in which n=0 to 2 in a total amount of 70 wt.% or greater based on all the sulfates;
(B) from 0.1 to 15 wt.% of a monoalkyl glyceryl ether or monoalkenyl glyceryl ether having a C₄₋₁₂ alkyl or alkenyl group; and
(C) from 0.1 to 10 wt.% of a silicone oil represented by the following formula (2):
R' (CH₃)₂SiO-[(CH₃)₂SiO]ₘ-Si(CH₃)₂R' (2)
(wherein, R' represents a methyl or hydroxy group and m is from 50 to 20000) and existing as disperse particles having an average particle size less than 50 µm.

2. The aqueous hair cleansing composition according to Claim 1, further comprising a nonionic surfactant or amphoteric surfactant other than Component (B).

3. The aqueous hair cleansing composition according to Claim 1 or 2, further comprising a cationic polymer.

4. The aqueous hair cleansing composition according to any one of Claims 1 to 3, further comprising a silicone other than Component (C).

5. The aqueous hair cleansing composition according to any one of Claims 1 to 4, further comprising a pearling agent containing an ethylene glycol monoalkyl ester or ethylene glycol dialkyl ester.

6. The aqueous hair cleansing composition according to any one of Claims 1 to 5, further comprising a higher alcohol.
